# FASCICULE DE BREVET EUROPEEN

(11) **EP 3 857 299 B1**
(45) Date de publication et mention de la délivrance du brevet: **13.12.2023**
(21) Numéro de dépôt: 19773827.1
(22) Date de dépôt: 24.09.2019
(51) Int. Cl.: G02C 13/00, A61B 3/11

(54) **INSTRUMENT DE MESURE POUR MESURER DES PARAMÈTRES NÉCESSAIRES À LA RÉALISATION DE LUNETTES**
MESSGERÄT ZUR MESSUNG DER FÜR DIE HERSTELLUNG EINER BRILLE NOTWENDIGEN PARAMETER
MEASURING INSTRUMENT FOR MEASURING PARAMETERS NECESSARY FOR PRODUCING SPECTACLES

(30) Priorité: 24.09.2018 FR 1858645
(43) Date de publication de la demande: 04.08.2021
(73) Titulaire: ESSILOR INTERNATIONAL, 94220 Charenton-Le-Pont (FR)
(72) Inventeur: WERMES, Jonathan, 75019 PARIS (FR); ENCAOUA, David, 78420 CARRIERES SUR SEINE (FR)
(74) Mandataire: Ipsilon
(86) Numéro de dépôt international: PCT/EP2019/075746
(87) Numéro de publication internationale: WO 2020/064758

(56) Documents cités:
- WO-A1-2014/006341
- FR-A1- 2 950 984
- FR-A1- 2 978 841
- US-A1- 2009 021 693
- US-A1- 2015 109 577

## Description

### DOMAINE DE L'INVENTION

L'invention concerne le domaine de la prise de mesures effectuées habituellement par un opticien en vue de la personnalisation de lentilles ophtalmiques correctrices et de leur montage sur une monture. Plus précisément, l'invention concerne le domaine de la métrologie optique, c'est-à-dire la mesure des différents paramètres nécessaires à la réalisation de lunettes.

### ARRIERE-PLAN TECHNOLOGIQUE

Les magasins d'optiques manquent habituellement de place : leurs murs et leurs espaces disponibles sont essentiellement occupés par des présentoirs de lunettes, des miroirs pour que les clients puissent essayer les lunettes ainsi que des écrans permettant de diffuser diverses informations, notamment des présentations de produits, à des fins souvent publicitaires ou pour faire patienter les clients. Ce manque de place est un obstacle majeur au déploiement d'un instrument de mesure pour mesurer des paramètres de vision d'un porteur, du type de l'instrument de mesure décrit dans le document FR2950984 ou la colonne ACTIVISU proposée par la Demanderesse. Une telle colonne prend en effet la place d'un présentoir, d'un miroir et/ou d'un écran.

Pour remédier à ce problème, la colonne ACTIVISU comprend par exemple un grand miroir sans tain, derrière lequel est placée la caméra. Le porteur peut alors se regarder dans le miroir pour essayer une monture ou être pris en photo, puis se voir dans le miroir une fois qu'il a remis ses lunettes correctrices. A côté de la colonne est fixé un écran qui sert au pilotage de la colonne et à différents contrôles et interactions pendant la prise de mesure. Lorsque la colonne n'est pas utilisée pour effectuer des mesures, l'écran peut diffuser des informations ou des animations, comme les autres écrans du magasin.

Cependant, cet écran est de petite taille en comparaison avec les écrans publicitaires et attire donc moins facilement l'attention. De plus, de par sa position sur la colonne, il masque une partie d'un présentoir à lunettes mural ou empêche la fixation d'un tel présentoir. D'autres instruments de mesure sont divulguées dans les documents WO2014/006341 A1, US2009/021693A1 et FR2978841A1.

Par ailleurs, lors des mesures pour la personnalisation des lentilles correctrices de lunettes et le centrage/montage de ces lentilles dans la monture, il est demandé au sujet de se placer devant le miroir et de regarder son reflet dans ce dernier. Pendant ces mesures, le sujet doit regarder droit devant lui (en visant globalement le pontet de sa monture dans le miroir ou le centre du visage, s'il ne distingue pas le pontet), à l'horizontale, à une distance comprise généralement entre trois et cinq mètres. Ce placement du porteur par rapport à l'instrument de mesure est important pour obtenir des mesures de qualité. Toutefois, il apparaît que les mesures obtenues sont souvent faussées en raison d'une mauvaise orientation droite-gauche du visage du porteur (erreur de mesure sur l'angle de cap), d'une intimidation du porteur liée au miroir ou à l'environnement et faussant la posture naturelle du porteur (erreurs de mesure sur l'angle pantoscopique et éventuellement de cap) ou d'une mauvaise orientation par rapport au déplacement pour arriver devant la colonne.

### RESUME DE L'INVENTION

Un objectif de l'invention est donc de proposer un instrument de mesure de paramètres nécessaires à la réalisation de lunettes correctrices capable de proposer une solution aux problèmes identifiés ci-dessus, et qui permettent en particulier d'aider le porteur à se positionner correctement par rapport à l'instrument de mesure, pendant les mesures, sans pour autant présenter un encombrement gênant pour le magasin d'optique ni réduire l'espace disponible pour les présentoirs de monture, les miroirs et les écrans publicitaires.

Pour cela, l'invention propose un instrument de mesure pour mesurer des paramètres nécessaires à la réalisation de lunettes correctrices pour un porteur comprenant :
- un bâti,
- au moins un écran au moins partiellement occultant et capable de réfléchir au moins partiellement la lumière, monté sur le bâti,
- au moins un écran, monté sur le bâti,
- au moins un dispositif de prise de vue, monté sur le bâti de sorte à être masqué par l'écran au moins partiellement occultant et
- des moyens de substitution réversibles de l'écran par l'écran au moins partiellement occultant de sorte à présenter l'écran au porteur pendant une phase de positionnement du porteur par rapport à l'instrument et de substituer à cet écran l'écran au moins partiellement occultant pendant une phase de prise de mesures.

Certaines caractéristiques préférées mais non limitatives de l'instrument de mesure sont les suivantes, prises individuellement ou en combinaison :
- l'écran au moins partiellement occultant comprend un miroir sans tain,
- le bâti comprend en outre un pied configuré pour maintenir l'instrument de manière stable sur un sol, une première face et une deuxième face distinctes, l'écran au moins partiellement occultant étant fixé sur la première face tandis que l'écran est fixé sur la deuxième face, et dans lequel les moyens de substitution comprennent une liaison pivot configurée pour mettre en rotation le bâti par rapport au pied autour d'un axe de rotation de sorte qu'une rotation du bâti autour de l'axe de rotation a pour effet de placer l'écran au moins partiellement occultant ou l'écran face au porteur.
- l'instrument de mesure comprend en outre au moins une butée et/ou un codeur configurés pour limiter une course du bâti autour de l'axe de rotation.
- les moyens de substitution comprennent des glissières configurées de sorte que l'un parmi l'écran au moins partiellement occultant ou l'écran est monté à coulissement sur le bâti de sorte à masquer ou à dévoiler l'autre parmi l'écran au moins partiellement occultant ou l'écran.
- l'instrument de mesure comprend en outre un dispositif de contrôle sans fil configuré pour envoyer des instructions au dispositif de prise de vue et à l'écran.
- l'instrument de mesure comprend en outre un espace de rangement formé dans le bâti et configuré pour recevoir le dispositif de contrôle.
- une hauteur de l'écran est comprise entre 30 % et 100 % d'une hauteur de l'écran au moins partiellement occultant.
- une hauteur du bâti est comprise entre 1.80 m et 2.20 m, une hauteur de l'écran au moins partiellement occultant est comprise entre 0.90 m et 2.0 m, de préférence entre 0.90 m et 1.60 m, et une hauteur de l'écran est comprise entre 0.30 m et 1.60 m

Selon un deuxième aspect, l'invention propose également un procédé de prise de mesure de paramètres nécessaires à la réalisation de lunettes correctrices à l'aide d'un instrument comme décrit ci-dessus, comprenant les étapes suivantes :
S1 : positionnement du porteur à une distance prédéfinie du bâti de l'instrument,
S2 : placement de l'écran face au porteur et affichage d'au moins une image,
S3 : placement de la monture des lunettes correctrices sur le visage du porteur,
54 : substitution de l'écran par l'écran au moins partiellement occultant,
S5 : prise des mesures.

Optionnellement, l'étape S4 est réalisée par rotation du bâti par rapport à un pied de l'instrument autour d'un axe de rotation ou par coulissement de l'un parmi l'écran ou l'écran au moins partiellement occultant.BREVE

### DESCRIPTION DES DESSINS

D'autres caractéristiques, buts et avantages de la présente invention apparaîtront mieux à la lecture de la description détaillée qui va suivre, et au regard des dessins annexés donnés à titre d'exemples non limitatifs et sur lesquels :
La figure 1 est une vue avant en perspective illustrant un exemple de réalisation d'un instrument de mesure conforme à un mode de réalisation de l'invention, le miroir sans tain étant présenté face au porteur.

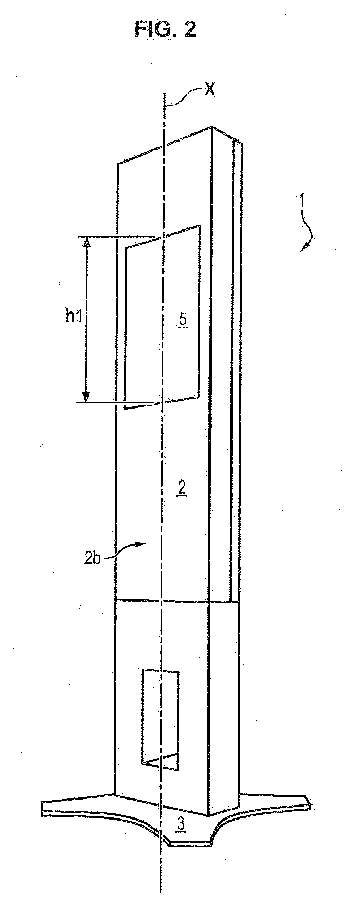

La figure 2 est une vue arrière en perspective de l'exemple d'instrument de mesure de la figure 1.
La figure 3 est une vue schématique sur laquelle sont représentés différents équipements possibles pour un instrument de mesure conforme à un mode de réalisation de l'invention, le miroir sans tain ayant été omis pour simplifier la lecture de la figure.

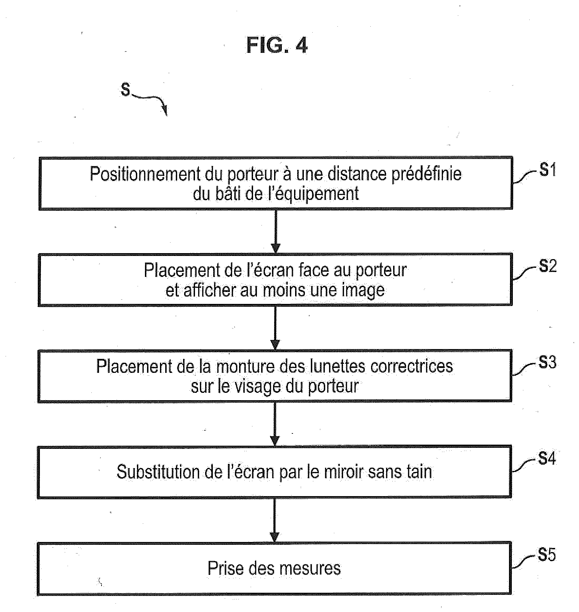

La figure 4 est un organigramme illustrant différentes étapes d'un procédé de prise de mesure de paramètres nécessaires à la réalisation de lunettes correctrices conforme à un mode de réalisation de l'invention et pouvant être mis en oeuvre à l'aide d'un instrument de mesure conforme à l'invention.
La figure 5 est une vue avant en perspective illustrant un deuxième exemple de réalisation d'un instrument de mesure conforme à un mode de réalisation de l'invention, le miroir sans tain étant présenté face au porteur.

### DESCRIPTION DETAILLEE D'UN MODE DE REALISATION

Un instrument 1 de mesure pour mesurer des paramètres nécessaires à la réalisation de lunettes correctrices d'un porteur comprend :
- un bâti 2 comprenant un pied 3 configuré pour être posé sur le sol et tenir debout, sensiblement verticalement, de manière stable,
- au moins un écran 4 au moins partiellement occultant capable de réfléchir au moins partiellement la lumière et au moins un écran 5, montés sur le bâti 2,
- au moins un dispositif de prise de vues 6, monté sur le bâti 2 de sorte à être masqué par le miroir sans tain 4.

Dans ce qui suit, l'invention sera décrite plus particulièrement ans le cas où l'écran 4 au moins partiellement occultant comprend un miroir sans tain. Ceci n'est cependant pas limitatif, l'écran 4 au moins partiellement occultant pouvant comprendre tout type d'écran capable de masquer le dispositif de prise de vues 6, tout en réfléchissant suffisamment la lumière pour que le porteur puisse voir son reflet dans l'écran. Par exemple, l'écran 4 peut comprendre tout semi-réfléchissant capable d'occulter au moins 50% de la lumière.

De manière connue en soi, le dispositif de prise de vues 6 peut comprendre une caméra vidéo, une caméra infrarouge, un appareil photographique numérique, un scanner ou tout autre moyen. De préférence, le dispositif de prise de vues 6 est configuré pour capturer des images dans le spectre visible (caméra couleurs par exemple). Ce dispositif de prise de vues 6 est placé dans la région supérieure du bâti 2, dans un plan de symétrie du bâti 2 (c'est-à-dire en étant globalement centré par rapport aux bords droite et gauche du bâti 2) de sorte à pouvoir être positionné facilement en face du visage du porteur.

Le cas échéant, afin d'adapter la position du dispositif de prise de vues 6 à la taille du porteur, celui-ci peut être monté mobile en translation sur une ou plusieurs glissières. Le déplacement du dispositif de prise de vues 6 le long des glissières peut être actionné par un moteur adapté, par exemple un moteur pas à pas afin d'obtenir un positionnement précis par rapport au visage du porteur. En variante, c'est tout ou partie du bâti 2 (par exemple sa partie supérieure sur lesquels le dispositif de prise de vues 6, le miroir sans tain 4 et l'écran 5 sont montés) qui est déplacé par un tel moteur le long de glissières de sorte à déplacer simultanément tous les équipements de l'instrument 1.

Optionnellement, l'instrument 1 peut en outre comprendre un frein électromécanique afin de maintenir le dispositif de prise de vues 6 en position après son déplacement par le moteur, ce qui permet de supprimer les courants de maintien.

Le miroir sans tain 4 est placé devant le dispositif de prise de vues 6, afin de permettre simultanément au porteur de se voir dans le miroir sans tain 4 et au dispositif de prise de vues 6 d'acquérir des images du porteur.

L'instrument 1 comprend en outre une unité de traitement comprenant des moyens de traitement, par exemple un ordinateur ou un serveur disposant de moyens de traitement et d'une mémoire, adapté pour exécuter le procédé S de prise de mesures qui sera décrit plus en détails ci-après. Les moyens de traitement peuvent par exemple comprendre un calculateur de type processeur, microprocesseur, microcontrôleur, etc.,

L'instrument 1 comprend également des moyens de commande 7 (écran tactile, clavier, souris, boutons, etc.). Dans une forme de réalisation, les moyens de commande 7 comprennent une tablette sans fil, et donc portable, équipée d'un écran 5 tactile pouvant être saisie par l'opérateur afin de piloter l'instrument 1 et contrôler les différents équipements qui le composent. La tablette 7 remplace avantageusement l'écran 5 de contrôle habituellement fixé sur le bâti 2 des instruments 1 de mesure conventionnels qui, en plus d'être encombrant et d'empiéter sur l'espace disponible pour les présentoirs à monture, pouvait gêner ou contraindre le porteur en imposant l'opérateur dans leur champ de vision.

Le cas échéant, un espace de rangement de la tablette 7 peut être prévu dans le bâti 2, par exemple dans une face latérale de celui-ci.

L'écran 5 quant à lui peut être du type écran à cristaux liquides, écran à DELs (pour diodes électroluminescentes) ou tout autre écran haute définition. De préférence, la hauteur h1 de l'écran 5 est comprise entre 30 % et 100 % de la hauteur h2 du miroir sans tain 4. Par « hauteur » d'un élément de l'instrument 1, on comprendra ici la distance suivant un axe sensiblement vertical, lorsque le bâti 2 est placé de manière stable sur le sol, entre une limite inférieure et une limite supérieure de l'élément. Typiquement, la hauteur h3 du bâti 2 peut être comprise entre 1.80 m et 2.20 m. La hauteur h2 du miroir peut alors être comprise entre 90 cm et 2.0 m, de préférence entre 90 cm et 1.60 m, tandis que la hauteur h1 de l'écran 5 peut être comprise entre 30 cm et 1.60 m. De par sa grande taille, l'écran 5 peut donc être utilisé comme outil de diffusion de publicité efficace et autorise une grande interactivité avec le porteur lors des phases de positionnement et de placement S1 à S3 (qui seront détaillées par la suite).

L'écran peut par ailleurs occuper tout ou partie d'une largeur du bâti 2.

Afin d'aider le porteur à se positionner correctement par rapport à l'instrument 1 de mesure, sans pour autant réduire l'espace disponible dans un magasin d'optique, l'instrument 1 comprend en outre des moyens de substitution 8 réversibles de l'écran 5 par le miroir sans tain 4 de sorte à présenter l'écran 5 au porteur pendant les phases de positionnement S1, de placement S2, S3 et en dehors de la prise de mesure, et de substituer à cet écran 5 le miroir sans tain 4 pendant la phase de prise de mesures S5.

De la sorte, le bâti 2 comprend une première configuration, dans laquelle l'écran 5 est présenté au porteur afin de détendre le porteur, de l'amener vers une posture naturelle et non contrainte par son environnement, d'effectuer des opérations préliminaires (entrée manuelle de données relatives au porteur ou à la monture, etc.). Par exemple, une animation présentant la suite de la procédure de prise de mesure et ce qui est attendu du porteur pendant cette procédure peut être affichée sur l'écran 5 à l'attention du porteur. Durant cette phase, le porteur peut porter ses lunettes correctrices, ce qui peut l'aider à améliorer son positionnement par rapport à l'écran 5 - et donc au miroir sans tain 4 et au dispositif de prise de vues 6.

Le bâti 2 comprend une deuxième configuration, dans laquelle le miroir sans tain 4 est présenté au porteur et les mesures sont effectuées, en substituant le miroir sans tain 4 à l'écran 5. Le porteur n'a pas besoin de se déplacer et doit simplement mettre la monture pour laquelle des mesures doivent être effectuées. Le cas échéant, un accessoire peut être placé sur la monture afin de faciliter les mesures. Le porteur est alors toujours dans une position confortable et les mesures peuvent être effectuées.

On notera que, grâce aux moyens de substitution 8, l'écran 5 peut avoir servi par exemple de support publicitaire avant le début de la prise de mesures. L'instrument 1 n'a donc pas empiété sur les zones disponibles dans le magasin d'optique.

Les moyens de substitution 8 sont en outre configurés pour substituer l'écran 5 au miroir sans tain 4 une fois la prise de mesures effectuée par l'opérateur (moyens réversibles).

Dans un premier mode de réalisation, la substitution réversible du miroir sans tain 4 à l'écran 5 peut être effectuée par rotation du bâti 2 autour d'un axe de rotation X, qui peut par exemple être sensiblement vertical.

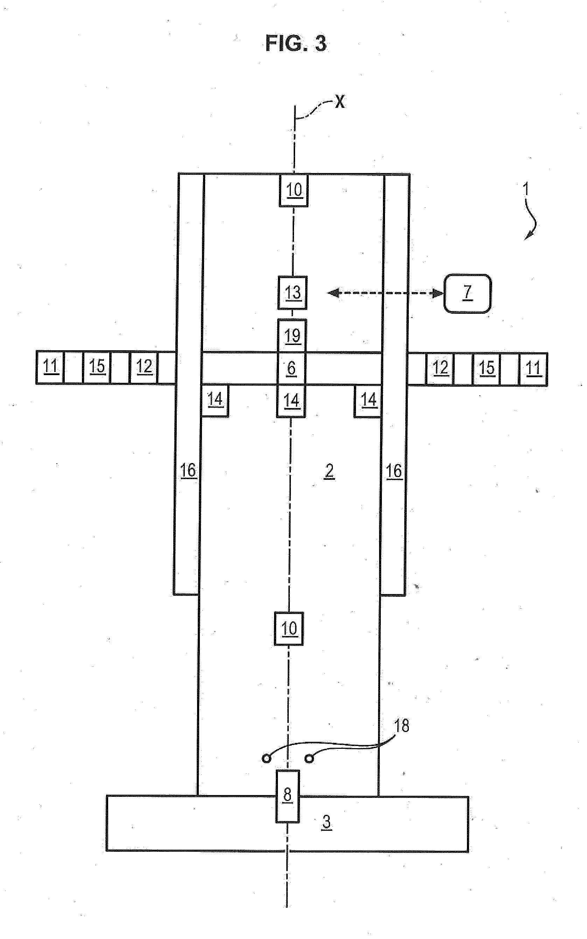

Dans ce mode de réalisation, le miroir sans tain 4 est alors fixé sur une première face 2a du bâti 2 tandis que l'écran 5 est fixé sur une deuxième face 2b du bâti 2, qui est distincte de la première face 2a. Par exemple, la première face 2a et la deuxième face 2b peuvent être sensiblement parallèles et opposées l'une à l'autre, de part et d'autre du bâti 2. Les moyens de substitution 8 comprennent alors une liaison pivot (voir figure 3) s'étendant entre le pied 3 de l'instrument 1 et le bâti 2 et configurée pour mettre en rotation le bâti 2 autour de l'axe de rotation X par rapport au pied 3 de sorte qu'une rotation du bâti 2 autour de l'axe de rotation X a pour effet de présenter l'une ou l'autre des faces du bâti 2, et donc de placer le miroir sans tain 4 ou l'écran 5 au porteur, sans risquer de déplacer l'instrument 1 par rapport au porteur. On notera en effet que la mise en rotation du bâti 2 en le soulevant et en le faisant tourner sur lui-même manuellement, ou en le faisant rouler sur le sol, présente le risque de fausser le positionnement du porteur par rapport à l'instrument 1 et donc de dégrader les mesures puisque l'opérateur ne peut pas garantir que le miroir a effectivement pris la même position par rapport au porteur que l'écran 5. Cela perturbe en outre le porteur, qui risque de modifier sa position pendant l'opération de déplacement de l'instrument 1 par l'opérateur.

Une telle substitution est en outre réversible, dans la mesure où il suffit de faire tourner le bâti 2 dans le sens inverse ou de poursuivre la rotation afin de replacer l'écran 5 en face du porteur.

Dans une forme de réalisation, l'instrument 1 comprend en outre un moteur, configuré pour mettre en rotation le bâti 2 autour de son axe de rotation X. Le moteur peut par exemple être du type pas à pas afin de permettre un positionnement précis du bâti 2.

En variante, le bâti 2 peut être monté libre de rotation autour de l'axe de rotation X. Dans ce cas, c'est donc l'opérateur qui fait tourner manuellement le bâti 2 afin de placer l'écran 5 et le miroir sans tain 4 dans la position adaptée.

Le cas échéant, quelle que soit la forme de réalisation, l'instrument 1 peut comprendre en outre au moins une butée et/ou un codeur configurés pour limiter la course du bâti 2 autour de l'axe de rotation X. La butée peut être placée de sorte à limiter la rotation du bâti 2 par rapport à son pied 3 à environ 360°, afin d'éviter d'endommager les câbles connectant par voie filaire les différents équipements de l'instrument 1 à leur alimentation respective ou à des moyens de traitement.

Optionnellement, le dispositif de prise de vues 6 est solidaire en rotation de l'écran au moins partiellement occultant.

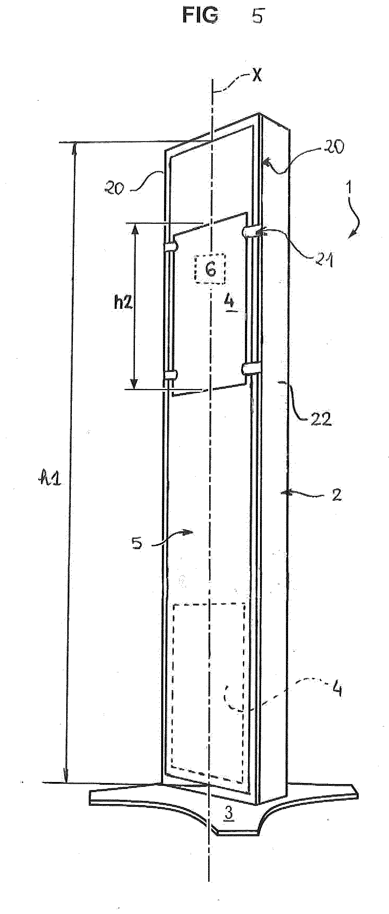

Dans un deuxième mode de réalisation illustré sur la figure 5, la substitution réversible du miroir sans tain 4 à l'écran 5 peut être réalisée par coulissement de l'un par rapport à l'autre.

Par exemple, l'écran 5 et le miroir sans tain 4 peuvent être fixés sur le bâti 2 de sorte à s'étendre sensiblement parallèlement l'un par rapport à l'autre. L'un parmi le miroir sans tain 4 et l'écran 5 - par exemple l'écran 5 ou selon la figure 5, le miroir sans tain - est alors monté mobile à translation par rapport à l'autre - par exemple le miroir sans tain 4 ou selon la figure 5, l'écran 5 - sur des glissières et le masque lorsqu'il est placé devant. De la sorte, la substitution du miroir sans tain 4 à l'écran 5 se fait simplement par translation de l'un des éléments (dans l'exemple, l'écran 5 ou selon l'exemple illustré, le miroir sans tain 4) par rapport à l'autre (le miroir sans tain 4 ou selon l'exemple illustré, l'écran 5) afin de permettre sa substitution sans avoir à déplacer l'instrument 1.

Le cas échéant, des charnières 20 peuvent être prévues en tête d'instrument 1 ou le long des tranches 22 du bâti 2 afin de permettre le basculement de l'élément monté à translation (par exemple, l'écran 5 ou selon l'exemple illustré, le miroir sans tain 4), de libérer toute la surface de l'élément fixe (dans l'exemple, le miroir sans tain 4) ou selon l'exemple illustré, une majeure partie de la surface de l'écran 5 selon l'exemple illustré et d'éviter de perturber la vision du porteur pendant les mesures
De préférence, l'écran 5 est monté mobile en translation sur des glissières, fixées sur le bâti 2 de part et d'autre du miroir sans tain 4, et masque au moins partiellement le miroir sans tain 4 lorsqu'il est placé devant. Cela évite en effet d'avoir à déplacer le dispositif de prise de vues 6 et son actionneur avec le miroir sans tain 4 lors de la substitution du miroir à l'écran 5.

Au contraire, selon la variante illustrée, c'est le miroir sans tain 4 qui est monté mobile en translation sur des glissières 20, fixées sur le bâti 2 de part et d'autre du miroir sans tain 4, par l'intermédiaire de moyens complémentaires 21 fixés sur les bords latéraux du miroir sans tain 4 et engagés dans les glissières de façon à assurer la translation du miroir sans tain 4 vis-à-vis du bâti. Le miroir sans tain 4 masque alors au moins partiellement l'écran 5 lorsqu'il est placé devant. Le fait de pouvoir placer le miroir sans tain 4 devant l'écran 5 permet à ce dernier de pouvoir bénéficier d'une hauteur h1 maximale (par exemple sensiblement celle de la colonne ou celle de la colonne à laquelle serait retranchée la hauteur du miroir sans tain 4).

On représente en pointillé sur cette figure la position inactive du miroir sans tain 4 dans laquelle il libère la majeure partie de l'écran 5 en étant disposé suite à sa translation le long des glissières, en partie basse du bâti. Dans cet exemple, l'écran 5 présente une hauteur sensiblement égale à celle de la colonne et son affichage pourra être dimensionné sur la hauteur visible de l'écran lorsque le miroir sans tain 4 occupe la position inactive.

Dans ce mode de réalisation où le miroir sans tain 4 est l'élément mobile, le dispositif de prise de mesure 6, peut soit être fixe vis-à-vis du bâti, ou mobile en translation verticale vis-à-vis du bâti et dans ce cas optionnellement rendu solidaire en translation du miroir sans tain 4.

L'on pourra en outre tirer profit de la mobilité en translation du miroir sans tain 4 et du dispositif de mesure 6, ainsi qu'éventuellement de l'un ou l'autre des ensembles formé par la source lumineuse infrarouge 14, les caméras infrarouge 15, et/ou les bras rotatifs, afin d'ajuster la hauteur de cet ensemble de mesure, à la taille du sujet concerné.

Optionnellement, le dispositif de prise de vues 6 est solidaire en translation de l'écran au moins partiellement occultant.

L'instrument 1 peut comprendre d'autres équipements permettant à l'opérateur d'effectuer diverses mesures de paramètres nécessaires à la réalisation de lunettes correctrices.

Ces paramètres comprennent, de manière connue en soi, l'un au moins des paramètres suivants : les demi-écarts pupillaires, la hauteur des yeux du porteur par rapport au bord inférieur de la lentille ou de la monture de lunettes, la distance verre-oeil, la position du centre de rotation de l'œil par rapport au verre, l'angle pantoscopique (qui correspond la mesure de l'inclinaison du plan moyen de la lentille correctrice autour d'un axe horizontal, par rapport à la verticale. Il s'agit d'un angle orienté qui reflète le fait que le sujet a tendance à avoir le visage plus ou moins relevé ou abaissé lorsqu'il regarde un objet placé droit devant lui), le galbe et l'angle de cap (qui correspond à la mesure de l'angle de rotation autour d'un axe vertical du plan formé par les deux segments naseaux de la monture. Le cap est un angle orienté qui reflète le fait que le sujet a tendance à avoir le visage plus ou moins tourné à gauche ou à droite lorsqu'il regarde un objet placé droit devant lui), les paramètres définissant la dimension des rectangles englobants des lentilles (cotes A et B) et leur position relative (cote D et galbe).

A cet effet, l'instrument 1 peut en outre comprendre l'un au moins des équipements suivants :
- au moins une cible visuelle 9, 10, 11, 12, 13,
- au moins une source lumineuse infrarouge 14 et au moins une caméra infrarouge 15,
- au moins un haut-parleur pour donner des instructions orales à l'utilisateur sans le divertir visuellement,
- au moins une bande d'éclairage 16,
- des moyens de marquage au sol 18.

L'au moins une cible visuelle peut comprendre une DEL émettant dans le spectre visible. Par exemple, l'instrument 1 peut comprendre :
- Une première cible visuelle 9, montée sur le bâti 2 derrière le miroir sans tain 4 et émettant des rayons lumineux en direction du visage du porteur à travers le miroir sans tain 4. De préférence, cette première cible visuelle 9 est solidaire en mouvement du dispositif de prise de vues 6 afin d'être positionnée sensiblement en face du visage du porteur.
- Deux deuxièmes cibles visuelles 10, montées sur le bâti 2 derrière le miroir sans tain 4, en partie supérieure et en partie inférieure du bâti 2 respectivement en étant sensiblement centrées sur celui-ci et sur le dispositif de prise de vues 6, et/ou deux troisièmes cibles visuelles 11, montées sur le bâti 2 de part et d'autre du miroir dans tain. Les deuxièmes et troisièmes cibles visuelles 11 peuvent chacune comprendre une DEL émettant dans le spectre visible. Elles peuvent notamment être utilisées de sorte à induire un déplacement spontané haut/bas ou droite/gauche de la tête du porteur afin de l'amener dans sa posture naturelle de référence, non contrainte par son environnement. Le cas échéant, l'instrument 1 peut comprendre une quatrième cible visuelle 12 du type DEL émettant dans le spectre visible, centrée sur le bâti 2 et permettant de ramener le visage du porteur face au bâti 2 entre deux diversions. On pourra notamment se référer au document FR 2 961 608 au nom de la Demanderesse pour plus de détails sur la détermination de la posture de référence naturelle d'un porteur et les équipements nécessaires à cette détermination. Optionnellement, les deux deuxièmes cibles visuelles 10 peuvent être fixées sur deux bras escamotables, montés sur le bâti 2, afin de les déporter par rapport au dispositif de prise de vues 6 et d'augmenter le déplacement du visage du porteur. Par exemple, les troisièmes cibles visuelles 11 peuvent être positionnées sur les bras de sorte qu'un angle entre l'axe du regard du porteur lorsqu'il fixe chaque troisième cible visuelle et l'axe de son regard lorsqu'il fixe la quatrième cible visuelle 12 soit de l'ordre de 20°. De préférence, les bras escamotables et la quatrième cible visuelle 12 sont solidaires en mouvement et mobiles en translation suivant l'axe vertical de sorte à ajuster leur hauteur en fonction de la taille du porteur. Le cas échéant, les bras escamotables peuvent être solidaires en mouvement du dispositif de prise de vues 6.

- Deux cinquièmes cibles visuelles 13, montées sur le bâti 2 de part et d'autre du miroir sans tain 4 et pouvant chacune comprendre une DEL émettant dans le spectre visible. Elles peuvent notamment être utilisées pour la détermination de la position du centre de rotation des yeux du porteur, à partir de ses reflets cornéens. On pourra notamment se référer au document WO 2016/075372 pour un exemple de détermination de la position du centre de rotation à partir des reflets cornéens. Le cas échéant, les deux cinquièmes cibles visuelles 13 peuvent être fixées sur les bras escamotables, entre le miroir sans tain 4 et la troisième cible visuelle. En variante, les deux troisièmes cibles visuelles 11 peuvent éventuellement être utilisées pour la détermination du centre de rotation des yeux du porteur, en lieu et place des cinquièmes cibles visuelles 13.
- Au moins une source lumineuse infrarouge 14, comprenant par exemple une DEL infrarouge, de préférence trois DELs infrarouge, alignées et placées à proximité du dispositif de prise de vues 6. Cette source lumineuse infrarouge 14 est associées à deux caméras infrarouges 15, fixées de part et d'autre du miroir sans tain 4, soit sur les bras escamotables, soit à proximité des bords du bâti 2. L'ensemble formé par la source lumineuse infrarouge 14 et les caméras infrarouges 15 peut alors être utilisé afin d'acquérir des images des lunettes du porteur et mesurer des paramètres, tels que les paramètres définissant la dimension des rectangles englobants des lentilles (cotes A et B) et leur position relative (cote D et galbe). Avantageusement, l'utilisation combinée d'un dispositif de prise de vues 6 dans le domaine visible et dans le domaine infrarouge permet de garantir l'acquisition d'images sur lesquelles sont clairement visibles les montures des lunettes, certaines montures étant transparentes aux infrarouges ou non visibles. sur les images prises par une caméra vidéo. Par exemple, les caméras infrarouges 15 peuvent être placées de sorte à former un angle de +10° et -10° par rapport à un plan de symétrie de l'instrument 1. Le cas échéant, la source lumineuse infrarouge 14 et les caméras infrarouges 15 peuvent être solidaires en mouvement du dispositif de capture d'images de sorte à ajuster leur hauteur en fonction de la taille du porteur.

De préférence, l'instrument 1 comprend une bande verticale d'éclairage 16 de part et d'autre du miroir sans tain 4, lesdites bandes 16 étant configurées pour éclairer le visage du porteur lors de la capture des images par le dispositif de prise de vues 6. Par exemple, les bandes verticales d'éclairage 16 peuvent comprendre des bandes de DELs montées chacune sur un volet 17 correspondant fixé à l'aide de charnières de part et d'autre du miroir sans tain 4 de sorte à pouvoir être ouvert ou fermé selon les besoins de l'opérateur. Chaque volet 17 peut être déplacé manuellement par l'opérateur, ou en variante par un moteur dédié.

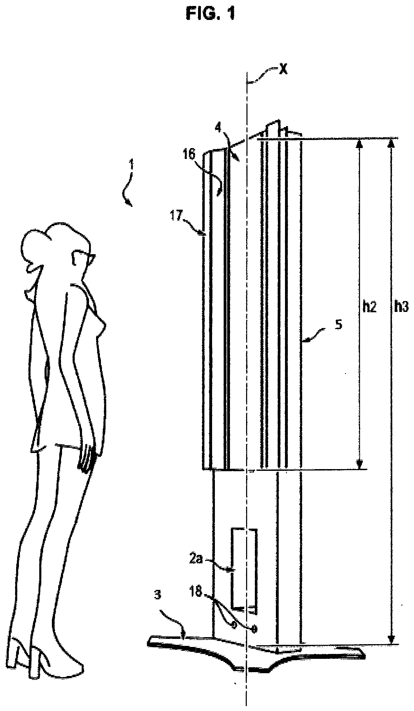

Les moyens de marquage 18 au sol peuvent comprendre au moins un laser, de préférence deux lasers, montés sur le bâti 2 et configurés de sorte à pointer vers une zone du sol correspondant à l'emplacement idéal du porteur par rapport à l'instrument 1. Ces moyens 18 permettent ainsi de garantir le positionnement du porteur et contribuent à la précision de la mesure afin que les images prises par le dispositif de prise de vues 6 et/ou les caméras infrarouges 15 aient une netteté optimale. Avantageusement, les moyens de marquage 18 sont fixés sur le bâti 2 de sorte à accompagner l'instrument 1 dans ses éventuels déplacements et à décaler automatiquement la zone du sol en fonction de la nouvelle position de l'instrument 1 (voir figures 1 et 3). Le cas échéant le ou les lasers peuvent être utilisés afin de projeter un logo ou une image marketing sur la zone du sol.

En variante, les moyens de marquage 18 comprennent un signe placé manuellement sur le sol par l'opérateur.

La prise de mesure de paramètres nécessaires à la réalisation de lunettes correctrices peut alors être réalisée conformément aux étapes suivantes :
S1 : positionnement du porteur à une distance prédéfinie du bâti 2 de l'instrument 1,
S2 : placement de l'écran 5 face au porteur et affichage d'au moins une image,
S3 : placement d'une monture sur le visage du porteur,
S4 : substitution de l'écran 5 par le miroir sans tain 4,
S5 : prise des mesures.

Plus précisément, au cours de la première étape S1, le porteur est placé à une distance optimale de l'instrument 1, et plus particulièrement du dispositif de prise de vues 6.

Par exemple, le porteur peut se placer sur la zone indiquée au sol par les moyens de marquage 18.

Au cours de la deuxième étape S2, l'écran 5 est placé face au porteur.

Généralement, l'écran 5 est déjà positionné de sorte à être face au porteur, puisqu'il peut être utilisé à des fins marketing dans le magasin d'optique. L'opérateur veille donc simplement à ce que l'écran 5 soit bien face au porteur, et non incliné par rapport à celui-ci.

En variante, l'instrument 1 peut être positionné de sorte que le porteur soit face au miroir sans tain 4. Dans ce cas, grâce aux moyens de substitution 8, l'opérateur substitue l'écran 5 au miroir sans tain 4, par exemple en faisant pivoter le bâti 2 autour de l'axe de rotation X jusqu'à ce que l'écran 5 se trouve face au porteur, ou en variante en faisant glisser l'écran 5 par-dessus le miroir sans tain 4.

Le porteur peut alors regarder l'écran 5 et se placer dans une posture confortable, sans être gêné par l'opérateur. En effet, l'opérateur pouvant contrôler l'instrument 1 à distance grâce à la tablette 7 tactile, il n'a pas besoin de se situer dans le champ de vision du porteur. De plus, la Demanderesse s'est aperçue du fait que le porteur pouvait être intimidé par son image dans le miroir sans tain 4, ce qui avait pour effet de fausser les mesures d'angle pantoscopique et de cap puisque le porteur n'était pas dans sa posture de référence.

Pendant cette phase, le porteur peut porter ses lunettes correctrices, de sorte à bien visualiser les images affichées sur l'écran 5, se détendre et venir naturellement dans sa posture de référence. En parallèle, l'opérateur peut observer le porteur, contrôler et éventuellement corriger la position de son visage et entrer des données préliminaires sur la monture ou le porteur. Les images projetées par l'écran 5 peuvent comprendre une animation expliquant le processus de mesure et ce qui est attendu du porteur pendant ces mesures.

Au cours de la troisième étape S3, le porteur retire ses lunettes correctrices et met la monture pour laquelle les paramètres doivent être mesurés. Cette étape est réalisée sans que le porteur ne se déplace ou ne bouge.

Le cas échéant, un accessoire peut être préalablement placé sur la monture afin de faciliter les mesures. Un exemple d'accessoire a par exemple été décrit dans le document FR 2 719 463 et comprend par exemple un support longiligne horizontal configuré pour être posé sur la bordure supérieure de la monture, une tige qui s'étend verticalement depuis le support et une partie en saille qui s'étend horizontalement, perpendiculairement au support et à la tige. Des cibles comprenant par exemple un marqueur coloré et/ou des formes géométriques sont fixées sur ces différentes parties de sorte que l'identification des cibles dans les images prises par le dispositif de prise de vues 6 permet, connaissant la position relative des cibles sur l'accessoire, de déterminer la configuration spatiale de la monture.

Au cours de la quatrième étape S4, qui peut être simultanée au préalable à la troisième étape S3, le miroir sans tain 4 est substitué à l'écran 5, par rotation autour de l'axe de rotation X ou par coulissement dans les glissières.

Au cours de la cinquième étape S5, les mesures des paramètres nécessaires à la réalisation des lunettes correctrices sont effectuées à l'aide du dispositif de prise de vues 6 et de tout ou partie des cibles visuelles 9-15 et/ou de tout ou partie des caméras infrarouges 15 et sources lumineuses infrarouges 14.

Par exemple, la première cible visuelle 9 peut être allumée afin que le porteur y focalise son regard, et une ou plusieurs images du visage du porteur sont acquises par le dispositif de prise de vues 6 et/ou par les caméras infrarouges 15. Des paramètres de vision du porteur peuvent alors être déterminés conformément au procédé décrit dans le document FR 2 860 887.

Le porteur peut en outre être amené dans sa posture de référence à l'aide des deuxièmes et troisièmes cibles visuelles 10, 11 et éventuellement de la quatrième cible visuelle 12, conformément au procédé décrit dans le document FR 2 961 608 mentionné ci-avant ou dans le document FR 2 950 984 au nom de la Demanderesse.

Le centre de rotation des yeux du porteur peut par ailleurs être déterminé à l'aide des cinquièmes cibles visuelles 13, conformément au procédé décrit dans le document WO 2016/075372 mentionné ci-avant.

Une fois les mesures effectuées, l'opérateur peut alors ranger la tablette 7 tactile dans l'espace de rangement ménagé dans le bâti 2.

## Revendications

1. Instrument (1) de mesure pour mesurer des paramètres nécessaires à la réalisation de lunettes correctrices pour un porteur comprenant :
- un bâti (2),
- au moins un écran au moins partiellement occultant (4), monté sur le bâti (2),
- au moins un écran (5), monté sur le bâti (2),
- au moins un dispositif de prise de vue, monté sur le bâti (2) de sorte à être masqué par l'écran au moins partiellement occultant (4),
l'instrument (1) étant **caractérisé en ce qu'**il comprend en outre des moyens de substitution (8) réversibles de l'écran (5) par l'écran au moins partiellement occultant (4) de sorte à présenter l'écran (5) au porteur pendant une phase de positionnement du porteur par rapport à l'instrument (1) et de substituer à cet écran (5) l'écran au moins partiellement occultant (4) pendant une phase de prise de mesures.

2. Instrument (1) selon la revendication 1, dans lequel le bâti (2) comprend en outre un pied (3) configuré pour maintenir l'instrument (1) de manière stable sur un sol, une première face (2a) et une deuxième face (2b) distinctes, l'écran au moins partiellement occultant (4) étant fixé sur la première face (2a) tandis que l'écran (5) est fixé sur la deuxième face (2b), et dans lequel les moyens de substitution (8) comprennent une liaison pivot configurée pour mettre en rotation le bâti (2) par rapport au pied (3) autour d'un axe de rotation (X) de sorte qu'une rotation du bâti (2) autour de l'axe de rotation (X) a pour effet de placer l'écran au moins partiellement occultant (4) ou l'écran (5) face au porteur.

3. Instrument (1) selon la revendication 2, comprenant en outre au moins une butée et/ou un codeur configurés pour limiter une course du bâti (2) autour de l'axe de rotation (X).

4. Instrument (1) selon la revendication 1, dans lequel les moyens de substitution (8) comprennent des glissières configurées de sorte que l'un parmi l'écran au moins partiellement occultant (4) ou l'écran (5) est monté à coulissement sur le bâti (2) de sorte à masquer ou à dévoiler l'autre parmi l'écran au moins partiellement occultant (4) ou l'écran (5).

5. Instrument (1) selon l'une des revendications 1 à 4, comprenant en outre un dispositif de contrôle (7) sans fil configuré pour envoyer des instructions au dispositif de prise de vue et à l'écran (5).

6. Instrument (1) selon la revendication 5, comprenant en outre un espace de rangement formé dans le bâti (2) et configuré pour recevoir le dispositif de contrôle (7).

7. Instrument (1) selon l'une des revendications 1 à 6, dans lequel une hauteur (h1) de l'écran (5) est comprise entre 30 % et 100 % d'une hauteur (h2) de l'écran au moins partiellement occultant (4).

8. Instrument (1) selon l'une des revendications 1 à 7, dans lequel une hauteur (h3) du bâti (2) est comprise entre 1.80 m et 2.20 m, une hauteur (h2) de l'écran au moins partiellement occultant (4) est comprise entre 0.90 m et 2.0 m, de préférence entre 0.90 m et 1.60 m, et une hauteur (h1) de l'écran (5) est comprise entre 0.30 m et 1.60 m

9. Procédé de prise de mesure de paramètres nécessaires à la réalisation de lunettes correctrices à l'aide d'un instrument (1) selon l'une des revendications 1 à 8 comprenant les étapes suivantes :
S1 : positionnement du porteur à une distance prédéfinie du bâti (2) de l'instrument (1),
S2 : placement de l'écran (5) face au porteur et affichage d'au moins une image,
S3 : placement de la monture des lunettes correctrices sur le visage du porteur,
S4 : substitution de l'écran (5) par l'écran au moins partiellement occultant (4),
S5 : prise des mesures.

10. Procédé selon la revendication 9, dans lequel l'étape S4 est réalisée par rotation du bâti (2) par rapport à un pied (3) de l'instrument (1) autour d'un axe de rotation (X) ou par coulissement de l'un parmi l'écran (5) ou l'écran au moins partiellement occultant (4).

## Patentansprüche

1. Messgerät (1) zur Messung von Parametern, die zur Herstellung einer Korrekturbrille für einen Träger erforderlich sind, beinhaltend:
- einen Ständer (2),
- mindestens einen mindestens teilweise undurchlässigen Schirm (4), der an dem Ständer (2) montiert ist,
- mindestens einen Bildschirm (5), der an dem Ständer (2) montiert ist,
- mindestens eine Bildaufnahmevorrichtung, die so an dem Ständer (2) montiert ist, dass sie durch den mindestens teilweise undurchlässigen Schirm (4) verdeckt wird,
wobei das Gerät (1) **dadurch gekennzeichnet ist, dass** es ferner reversible Mittel (8) zum Ersetzen des Bildschirms (5) durch den mindestens teilweise undurchlässigen Schirm (4) beinhaltet, um dem Träger während einer Positionierungsphase des Trägers in Bezug auf das Gerät (1) den Bildschirm (5) zu präsentieren und diesen Bildschirm (5) während einer Messaufnahmephase durch den mindestens teilweise undurchlässigen Schirm (4) zu ersetzen.

2. Gerät (1) nach Anspruch 1, wobei der Ständer (2) ferner einen Fuß (3), der dazu konfiguriert ist, das Gerät (1) stabil auf einem Boden zu halten, eine erste Seite (2a) und eine davon verschiedene zweite Seite (2b) beinhaltet, wobei der mindestens teilweise undurchlässige Schirm (4) auf der ersten Seite (2a) befestigt ist, während der Bildschirm (5) auf der zweiten Seite (2b) befestigt ist, und wobei die Mittel zum Ersetzen (8) eine Schwenkverbindung beinhalten, die dazu konfiguriert ist, den Ständer (2) in Bezug auf den Fuß (3) um eine Drehachse (X) in Drehung zu versetzen, sodass eine Drehung des Ständers (2) um die Drehachse (X) dazu führt, dass der mindestens teilweise undurchlässige Schirm (4) oder der Bildschirm (5) gegenüber dem Träger platziert wird.

3. Gerät (1) nach Anspruch 2, das ferner mindestens einen Anschlag und/oder einen Geber beinhaltet, die dazu konfiguriert sind, eine Bewegung des Ständers (2) um die Drehachse (X) zu begrenzen.

4. Gerät (1) nach Anspruch 1, wobei die Mittel zum Ersetzen (8) Gleitschienen beinhalten, die so konfiguriert sind, dass einer von dem mindestens teilweise undurchlässigen Schirm (4) oder dem Bildschirm (5) verschiebbar an dem Ständer (2) montiert ist, sodass er den anderen von dem mindestens teilweise undurchlässigen Schirm (4) oder dem Bildschirm (5) verdeckt oder freigibt.

5. Gerät (1) nach einem der Ansprüche 1 bis 4, das ferner eine drahtlose Steuervorrichtung (7) beinhaltet, die dazu konfiguriert ist, Anweisungen an die Bildaufnahmevorrichtung und an den Bildschirm (5) zu senden.

6. Gerät (1) nach Anspruch 5, das ferner einen Stauraum beinhaltet, der in dem Ständer (2) gebildet ist und dazu konfiguriert ist, die Steuervorrichtung (7) aufzunehmen.

7. Gerät (1) nach einem der Ansprüche 1 bis 6, wobei eine Höhe (h1) des Bildschirms (5) zwischen 30 % und 100 % einer Höhe (h2) des mindestens teilweise undurchlässigen Schirms (4) beträgt.

8. Gerät (1) nach einem der Ansprüche 1 bis 7, wobei eine Höhe (h3) des Ständers (2) zwischen 1,80 m und 2,20 m beträgt, eine Höhe (h2) des mindestens teilweise undurchlässigen Schirms (4) zwischen 0,90 m und 2,0 m, vorzugsweise zwischen 0,90 m und 1,60 m, beträgt und eine Höhe (h1) des Bildschirms (5) zwischen 0,30 m und 1,60 m beträgt.

9. Verfahren zur Messaufnahme von Parametern, die zur Herstellung einer Korrekturbrille erforderlich sind, mit Hilfe eines Geräts (1) nach einem der Ansprüche 1 bis 8, beinhaltend die folgenden Schritte:
S1: Positionieren des Trägers in einem vordefinierten Abstand zu dem Ständer (2) des Geräts (1),
S2: Platzieren des Bildschirms (5) gegenüber dem Träger und Anzeigen mindestens eines Bildes,
S3: Platzieren des Korrekturbrillengestells auf dem Gesicht des Trägers,
S4: Ersetzen des Bildschirms (5) durch den mindestens teilweise undurchlässigen Schirm (4),
S5: Aufnehmen der Messungen.

10. Verfahren nach Anspruch 9, wobei der Schritt S4 durch eine Drehung des Ständers (2) in Bezug auf einen Fuß (3) des Geräts (1) um eine Drehachse (X) oder durch eine Verschiebung von einem von dem Bildschirm (5) oder dem mindestens teilweise undurchlässigen Schirm (4) erfolgt.

## Claims

1. Measurement instrument (1) for measuring parameters necessary for producing corrective spectacles for a wearer, and comprising:
- a stand (2),
- at least one at least partially occluding screen (4) mounted on the stand (2),
- at least one screen (5), mounted on the stand (2),
- at least one image-capturing device, mounted on the stand (2) in such a way as to be hidden by the at least partially occluding screen (4),
the instrument (1) being **characterized in that** it further comprises reversible substitution means (8) for substituting the at least partially occluding screen (4) for the screen (5) so as to present the screen (5) to the wearer during a phase of positioning of the wearer with respect to the instrument (1) and for substituting the at least partially occluding screen (4) for this screen (5) during a measurement-taking phase.

2. Instrument (1) according to Claim 1, wherein the stand (2) further comprises a base (3) configured to keep the instrument (1) stable on the ground, a first face (2a) and a second face (2b), which are distinct, the at least partially occluding screen (4) being fixed to the first face (2a) while the screen (5) is fixed to the second face (2b), and wherein the substitution means (8) comprise a pivot connection configured to rotate the stand (2) with respect to the base (3) about an axis of rotation (X) so that rotating the stand (2) about the axis of rotation (X) has the effect of placing the at least partially occluding screen (4) or the screen (5) so that it faces the wearer.

3. Instrument (1) according to Claim 2, further comprising at least one end stop and/or an encoder which are configured to limit a travel of the stand (2) about the axis of rotation (X).

4. Instrument (1) according to Claim 1, wherein the substitution means (8) comprise slideways which are configured in such a way that one of either the at least partially occluding screen (4) or the screen (5) is mounted with the ability to slide on the stand (2) so as to hide or uncover the other of either the at least partially occluding screen (4) or the screen (5).

5. Instrument (1) according to one of Claims 1 to 4, further comprising a wireless control device (7) configured to send instructions to the image-capturing device and to the screen (5).

6. Instrument (1) according to Claim 5, further comprising a storage space formed in the stand (2) and configured to accept the control device (7).

7. Instrument (1) according to one of Claims 1 to 6, wherein a height (h1) of the screen (5) is comprised between 30% and 100% of a height (h2) of the at least partially occluding screen (4).

8. Instrument (1) according to one of Claims 1 to 7, wherein a height (h3) of the stand (2) is comprised between 1.80 m and 2.20 m, a height (h2) of the at least partially occluding screen (4) is comprised between 0.90 m and 2.0 m, preferably between 0.90 m and 1.60 m, and a height (h1) of the screen (5) is comprised between 0.30 m and 1.60 m.

9. Method for taking measurements of parameters necessary for producing corrective spectacles using an instrument (1) according to one of Claims 1 to 8, comprising the following steps:
S1: positioning the wearer at a predefined distance from the stand (2) of the instrument (1),
S2: placing the screen (5) so that it faces the wearer and displaying at least one image,
S3: placing the frame of the corrective spectacles on the wearer's face,
S4: substituting the at least partially occluding screen (4) for the screen (5),
S5: taking measurements.

10. Method according to Claim 9, wherein step S4 is performed by rotating the stand (2) with respect to a base (3) of the instrument (1) about an axis of rotation (X) or by sliding one of either the screen (5) or the at least partially occluding screen (4).
